# EUROPEAN PATENT APPLICATION

(11) **EP 1 652 528 A1**
(43) Date of publication of application: **03.05.2006**
(21) Application number: 04701884.1
(22) Date of filing: 14.01.2004
(51) Int. Cl.: A61K 33/44, A61K 9/08, A61P 35/00

(54) **ACTIVATED CARBON INFUSION SOLUTION, PREPARATION METHOD THEREFOR AND USE THEREOF FOR THE MANUFACTURE OF DRUG FOR TREATING CANCER**

(30) Priority: 06.05.2003 CN 03125090
(71) Applicant: Chen, Xiaochuan, Xiangtan, Hunan 411100 (CN); Wang, Pulin, Tangshan, Hebei 063000 (CN)
(72) Inventor: Chen, Xiaochuan, Xiangtan, Hunan 411100 (CN); Wang, Pulin, Tangshan, Hebei 063000 (CN)
(74) Representative: Kroher, Jürgen
(86) International application number: PCT/CN2004/000045
(87) International publication number: WO 2004/098620

(57) **Abstract**

The invention discloses activated carbon infusion solution, preparation method therefor and use thereof for the manufacture of drug for treating cancer. The said activated carbon infusion solution contains pure activated carbon microparticles injection solution, activated carbon microparticles injection solution, activated carbon injection solution adsorbing catalyst and various injection solutions carrying drugs, all of which are for intravenous use. It characterizes that the surface area of material activated carbon used is greater than or equal to 400-10000 M²/g or higher, heavy metal content is less than or equal to 0.1-10 ppm, other dissoluble metal ion content is less than or equal to 0.1-10 ppm, ash content is less than 2 %, total pore volume is greater than 0.3-6 cm²/g, 0.15 % methylene blue adsorptive value is greater than 6-30, the diameter of the activated carbon microparticles contained in this activated carbon infusion solution are ranged from 35 µm to 2 nm, the diameters are mainly ranged from 6 µm to 2 nm, in which 99 % are ranged from 3 µm to 2 nm, the microparticles which diameters are 6 µm to 3µm are not more than 1 %, the microparticles which greatest diameter is 35 µm to 6 µm have not more than 2 × 10⁴/mg activated carbon micropowder. When this activated carbon microparticles are intravenously injected into blood, they have good compatibility with tissues, have no toxicity or side effect, have no harmful stimulation, have no immunogenicity, they are safe and effective. They have incredible curative effect in treating cancer, blood vessels atherosclerosis, coronary heart disease, cerebral thrombosis, infectious diseases, azotemia, acute organic and inorganic poison poisoning.

## Description

### Field of the invention

The present invention relates to an activated carbon based transfusion agent, preparation thereof and use thereof for the manufacture of anticancer drugs. In particular, the present invention relates to an activated carbon agent used for intravenous transfusion, the corresponding preparation process and use thereof for manufacture of anticancer drugs.

### Background of the invention

Activated carbon has been used in medicine field for almost one hundred years. Generally, activated carbon is employed in purification process of manufacturing traditional Chinese and Western injections, such as those disclosed in the following documents: CN1377689, CN1287838, CN1068958, and CN1062083, where the referred activated carbon is filtered out after adsorption of the impurities or residue. Hence the finished injection products contain no activated carbon. It has been proved, by some medical experiments, that activated carbon has curative effect on a variety of diseases. For example, by way of oral administration, activated carbon agent can be used as curative against gastric illness; it can adsorb toxin in the alimentary tract such as hypnotic-induced toxicosis and prevention of drunkenness; alternatively, it can be employed as a urine sorbent for the assay of the athletes' exhilarant level; applied to the surface of festers, activated carbon will adsorb poison and stink; Application EP 620 006 disclosed therapeutic effect of activated carbon agent on diabetes; JP 11-029485 illustrated that activated carbon is curative against adiposity; US-4,152,412 dealt with activated carbon (0.5 micron) adsorbed vaccine, antigen and toxoid for animals use. This dosage form of the agent is released slowly and the corresponding efficacy of immune vaccine can be improved; US-1,465,052 provided an approach concerning the mixture of activated carbon with curatives for prevention and treating the ascarid in animals. By way of subcutaneous injection, this dosage form of the agent can enhance the curative efficiency and extend the drugs' activities by at least three months; WO 9108776 depicted the injection of 1-6.8 µm non-active or low-activated carbon particulate into cancer, thereby to mark cancers before the execution of chemotherapies and operations, as well as to absorb energy in case of laser or electromagnetic wave therapies. In this method, the carbon is injected in the vicinity of cancer tissue and takes effect as the controlled release of the drug. So the carbon particulate's function is to keep sustained release of the drugs in the intravascular and extravascular tissues. This method is an extension of external application principle, which essentially differs from the present invention as to the application approach and the curative mechanism, where activated carbon is directly transfused into blood. Based on long-term studies, activated carbon with large specific surface area and with a particles size between 6 µm - 2 nm shows extraordinary therapeutic efficiency on various cancers, atherosclerosis, coronary heart disease, cerebral thrombosis, infectious diseases, azotemia, acute organic and inorganic toxicosis, etc., by way of transfusion into blood or into abdominal cavity and thorax cavity together with the traditional intravenous transfusion fluid. A great deal of animal experiments suggest that, after transfused into blood, the activated carbon of the present invention shows perfect histocompatibility and has no toxic side effect, no negative irritation, and no immunogenicity. In addition, it is safe and effective without causing embolism and pathological damage.

Results of animal experiments also showed some tiny insoluble substances (rigid particles) with a grain size between 6 µm - 2 nm, such as gelose granule, glucan granule, cellulose granule, even the water-insoluble substances, such as the diatomite granule, zeolite granule, bentonite granule, are able to inhibit the growth of cancers and work as drug-carriers, but their security and therapeutic efficiency are obviously inferior to those of activated carbon.

### Description of the invention

It is one objective of the present invention to provide an activated carbon based transfusion agent, which is an activated carbon agent used for intravenous transfusion.

It is a further objective of the present invention to provide a method with unique arts and easy manipulation, for preparation of the activated carbon based intravenous transfusion agent.

It is yet a further objective of the present invention to encompass the pharmaceutical application of the activated carbon based intravenous transfusion in the manufacture process of anticancer drugs.

The activated carbon based transfusion agent of the present invention includes pure activated carbon particles injection, enabled activated carbon particles injection, activated carbon injection adsorbed with catalyst, and therapeutic drug-delivery activated carbon injection used for intravenous transfusion, the therapeutic drug-delivery activated carbon injections include those adsorbing anticancer drugs, anticoagulation and cholesterol-lowering drugs, antibiotics, and antivirus drugs, which is characterised in that the raw material, activated carbon, used in preparation of this group of agents should be in accordance with the following requirements: the specific surface area ≥400-10000 M²/g or even much larger, the content of heavy metals ≤0.1-10 ppm, other dissoluble metal ions ≤ 0.1-10 ppm, ash content < 2 %, the overall pore volume > 0.3-6 cm³/g, 0.15 % methylene blue adsorption > 6-30, the diameter of the activated carbon particles contained in this activated carbon based transfusion agent is from 35 µm to 2 nm, with particles size mainly distributed in a range of 6 µm -2 nm, of which those between 3 µm -2 nm account for 99 %, those between 6 µm -3 µm no more than 1 %, and those with largest size between 35 µm - 6 µm are counted no more than 2 x 10⁴/mg_{.}

Supported by a great deal of animal experiments, after infused into blood, the activated carbon of the present invention shows perfect histocompatibility and has no toxic side effect, no negative irritation, no immunogenicity. In case of normal dose (i.e., effective therapeutic dose of 0.1-5 mg/ body weight per time of transfusion into blood, 1-7 times per week, the total average cumulative dose for adults less than 10 g), within the range of preparation quality standard, the activated carbon will not cause vital embolism. The agent can be iteratively transfused into veins without any poisoning effect or vital damage, on condition that the therapeutic dose and total cumulative dose are finely controlled. Confirmed by animal anatomical results, transfusion of this type of activated carbon particles will not cause pathological damage to heart, liver, kidney, spleen, lung, bone medulla and cerebrum.

The features of the preparation method of the present invention lie in the following steps it encompasses:
(1) The activated carbon powder is physically ground into coarse 120 mesh particles;
(2) After coarse particles adsorbed the activator with concentration of 0.01-10 %, the activated carbon is activated. The reaction system is heated to 70-190°C in a high-pressure container for 5-20 minutes, resulted in oxidization-type or reduction-type activated carbon, cooled and decompressed the pressure of system, and discharge of the residue gas. Then aqueous vapor and ammonia vapor with temperatures at 60-100°C are forced into the system respectively and activated carbon comprising carbo-hydroxyl group, carbo-imino group or carbo-hydro group is then produced.
(3) As for drug-delivery treatment of the activated carbon, relevant remedy is selected to process the liquid saturated adsorption with enabled activated carbon or non-enabled pure activated carbon. Then it is desiccated under low temperature and smashed into activated carbon particles with preferred particles size by a jet pulverizer.
(4) The activated carbon particles are treated with dipping approach for adsorption of biological orienting agents, and then processed by rapid, deep-freeze desiccation.

The mentioned activator in step (2) can be divided into oxidant and reductant, wherein oxidant is selected from the group of epoxy aether, acyl chloride, anhydride, aldehyde, and reductant is selected from the group of LiAlH₄ and NaBH₄.

The relevant remedies mentioned in step (3) include: A) traditional anticancer drugs, such as 5-FU, CTX, MTX, Ara-C/A, 6-MP, anticancer antibiotics, cisplatin/carboplatin, arsenic, pacilitaxel, etc.; B) immune activating and regulating cytokines, such as: IL-2, 4, 6, GM, G-CSF, γ-IFN, TNF, FCF-B, VECF, 4-IBB/4-IBBL, B7-1B7-2, toad-poison protein, specific antihapten antibody, etc.; C) catalysts, such as core metal complexes with benzyl/ butyl lithium group, and transition metals including cuprammonium agent, platinum, palladium agent, selenium, germanium compounds and so on. These compounds are featured with double functions of catalysis and cancer-killing; D) absorbent biological enzymes, such as oxidoreductases, transaminases, hydrolases and isomerases etc.

The referred biological orienting agents in step (4) include ferroporphyrin, or a large group of thymine cytosine, uracil adenine, guanine, and their derivatives, such as 5- fluorouracil, hydrodeuteradenine, methotrexate, as well as cancer cell adhesion factors (e.g., vascular cell adhesion molecule-1, VCAM-1) and cancer cell growth factors (e.g., vascular enthothelial growth factor, VEGF).

Different remedies and biological orienting agents from step (3) and step (4) can be selected to prepare various therapeutic drug-delivery activated carbon agents, such as anticancer injections, absorbing anticoagulation and cholesterol-lowering drugs injections, antibiotics, and antivirus drugs.

The present invention further provides the application of the activated carbon based transfusion agents in the manufacture process of anticancer drugs. This application is characterized by effective therapeutic dose of 0.1-5 mg/ body weight per time of transfusion into blood, 1-7 times per week, the total average cumulative dose for adults less than 10 g.

Long-term studies prove that the activated carbon with diameter between certain range of nanometers - micrometers can remarkably alleviate cancer pain, inhibit cancer growth or execute cancer cells. By way of intravenous transfusion, the activated carbon particles can be employed to treat a variety of cancers, and atherosclerosis, coronary heart disease, cerebral thrombosis; the activated carbon particles, after infused into veins, also shows some immune factors like activities, such as IgG\A\M, DC small peptide molecules and immunogloblin super-family like activities, and hence can be used for treatment of various intractable, microbial and viral infections; In addition, it was also found that this activated carbon particles, after infused into veins, can promote the tissue reparation, cure chronic unhealed wounds and osteomyelitis with extraordinary therapeutic effect, and treat acute organic and inorganic toxicosis.

Beside the anticancer activity per se, pure activated carbon particles further permit absorption, immune regulation chemical catalysis and drug delivery. If continuously treated with chemical activation, drug-loading and orientation adsorption agent, the above mentioned activities will be positively enhanced and exceptional therapeutic effect will be achieved. This is the mechanism how the activated carbon transfusion agent is applied in pharmaceutical preparation of anticancer drugs. The detailed description of this embodiment of the present invention is depicted below:
1. Further activation of the pure activated carbon particles:
   The activated carbon is chemically catalytic active per se, in the meantime it may be modified with various chemical groups to enable it to combined with various chemical catalyst groups, such as low molecular compounds bearing hydroxyl group, carboxyl group, amino group, carbonyl and aryl group; chemical catalyst groups combined with the activated carbon can be enzymes organic compounds, or core metal organic compounds, transition metal organic compounds or corresponding inorganic compounds which can work in solution catalytically under normal temperature and pressure, thus permits the catalysis for hydrogen, oxygen, hydrocarbon, peptide and lipid; absorption capacity of activated carbon is increased evidently after being treated with chemical activation, and consequently, therapeutic catalysis of the activated carbon will become more pertinent, or drug delivery capacity will be upgraded.
2. Further drug-loading and the treatment using orientating adsorption agent for the enabled activated carbon particles:
   To combine biological orienting substances to the activated carbon particles is an effective method to further improve activated carbon particles transfusion therapy. Principles of selecting such orienting substances involve: to select orienting substances with uptakes for growth purpose in cancer cell higher than those in normal cells in view of the growth flourish of cancer cells, that is, to adopt orienting substances with high cancerphilic activity and without immunogenicity, such as base pairs indispensable for constructing DNA or RNA (a large group of thymine cytosine, uracil, adenine, guanine, and their derivatives), substances with uptake for metabolism purpose higher in cancer cells than that in normal cells by several hundred times (e.g., ferroporphyrin); or to choose substances featured by cancer affinity, such as cancer cell adhesion factors (e.g., vascular cell adhesion molecule-1, VCAM-1) and cancer cell growth factors (e.g., vascular enthothelial growth factor, VEGF). All the substances mentioned above are able to facilitate cancer growth. It is very interesting to find that cancer cells fancy uptaking of such activated carbon particles and engulf a mass of activated carbon particles until death and halt of growth occur, whereas normal tissue cells pay no or little attention to these particles. Thereby, the activated carbon particles with cancer cancerphilia and cancer affinity automatically congregate in the vicinity of cancer cells. Sequentially, the content of activated carbon particles within the cancer tissue is much higher than that of other normal tissues by several hundred, even up to ten thousand times. The creative combination of orienting substances and activated carbon breaks though the principle of biological orienting substances selection and settles the weak drug selectivity of cancer chemotherapy as well as the resistance against orienting substances.

According to various studies, within the cancer tissue characterized by very abundant and disorganized neovasculars, the activated carbon particles with cancer cancerphilia and cancer affinity or pure activated carbon particles will accumulate to a extremely high content in microvasculars or the dead ends of neomicrovasculars of cancer tissue and be engulfed in large quantity by cancer cells; a great deal of activated carbon was collected within cancer tissue and exert its function of release of anticancer drugs, immune regulation, chemical catalysis and absorption substance homeostasis to interfere the growth of cancer tissue, thereby work to kill cancer comprehensively.

Principles for selection of another antivirus orienting agent and drug delivery are: virus affinity agents in combination with virus disruptors, such as, thymine for DNA virus, uracil for RNA virus may be adopted to combine with interferon, IL-1, virus transcriptase inhibitors and polymerase inhibitors.

The activated carbon particles with biological orienting activity, because of their tremendous specific surface area, are able to adsorb manifold anticancer drugs, which congregate surrounding cancer cells along with the orientation of activated carbon. The carried drugs have various options, such as chemotherapy drugs, radioactive nuclides, immune regulating cytokines and the metal complex catalysts, such as those associated with platinum, palladium, copper, germanium, selenium, cobalt and so on. These drugs are orientated to cancer cells and within the microvasculars and extracellular matrix of cancer, congregate to a concentration of several hundred, even ten thousand times higher than that of routine therapies. In result, anticancer effect gains reinforcement and toxic side effect of chemotherapy drugs is decreased. Usually, the chemotherapy and radiotherapy drugs are able to reach or surpass traditional therapy in efficiency, with a dosage of 5-20 % of that of traditional therapy. In this way, damage of anticancer drugs to normal body cells and to immune system will be maximally lightened.

Animal experiments on security of the activated carbon of the present invention are described as following:
1. Animal toxicity experiment: 12 rats, with the body weight between 135 g ± 10 g, were adopted in this experiment. Half of them were male and half female. After injected with activated carbon via the tail vein, the rats were kept under observation for 28 days.

| Group | Dose (mg/kg) | No. of animals | Mortality | LD₅₀ | Cumulation (mg/kg) |
|---|---|---|---|---|---|
| A | 0.2 2/W x 5 | 12 | 0 | unobserved | 2 |
| B | 0.4 2/W x 5 | 12 | 0 | | 4 |
| C | 1.62/W x 5 | 12 | 0 | | 16 |
| D | 1.6 2/W x 16 | 12 | 0 | | 25.6 |
| E | 30 | 6 | 0 | | 30 |

Due to the difficulty of continuous injections via the tail vein, the rats were injected at most 2/W times. In the above table, Group D was identified as maximal tolerance test and group E expedient limit test.
Group A, B, C were about respectively low, moderate, high agent load involved in this animal acute toxicity experiment. They differed from acute toxicity experiment by more injection times and more days under observation.
2. Results of pathological anatomy: 2 rats were randomly sampled to undergo pathological anatomy. The results are summarized blow:

| Group | No.of animals | Heart | Liver | Lung | Spleen | Kidney | Encephalon | Spinal cord | Muscles | Ribs | Ventroiliac spine | Enteric lymph nodes | Thymus |
|---|---|---|---|---|---|---|---|---|---|---|---|---|---|
| A | 4 | No abnormal pathological findings observed | | | | | | | | | | | |
| B | 4 | No abnormal pathological findings observed | | | | | | | | | | | |
| C | 4 | No abnormal pathological findings observed | | | | | | | | | | | |
| D | 4 | No abnormal pathological findings observed | | | | | | | | | | | |
| E | 4 | Small patches of congestion found on exterior of one rat's lung, but without haemorrhagic pathological changes. No other abnormal pathological findings observed | | | | | | | | | | | |

| | | | | | | | | | | | | | |
|---|---|---|---|---|---|---|---|---|---|---|---|---|---|
| Note: The above illustrated animal experiments were some observed results from our study. They did not strictly meet the criteria of acute or chronic toxicity experiments dictated in the pharmacopoeia. For example, the administration doses for rats were higher than that requested by standard experiments; the observation time was also longer than that requested by standard experiments; the number of rats adopted in the experiment was less than that requested. | | | | | | | | | | | | | |

### Experimental clinical cases

### Case 1:

A male patient, aged 57 years old, was diagnosed with pulmonary cancer. Right supraclavicular lymph node biopsy revealed adenocarcinoma. He underwent several periods of chemotherapies, and was admitted this time with presentation of respiratory symptoms including chest distress, palpitation, tachypnea, and inability to lie down. Cardiac B ultrasound examination and chest computed tomography showed serious pericardiac effusion. The patient must sit all through 24 hours with a heart rate 120 beats/min. Attempt for extraction of pericardiac effusion failed because of the patient's unendurance. After intravenous injection of 20 mg activated carbon agent of the present invention, symptoms of distress, palpitation, tachypnea were alleviated 24 hours later. The patient was also able to lie down with heart rate of 90 beats/min. 48 hours later, cardiac B ultrasound for the second time suggested complete disappearance of pericardiac effusion.

### Case 2:

Another male patient, aged 21 years old, was diagnosed with alveolar cell carcinoma and his symptoms did not relieve after chemotherapy. Chest radiograph showed floccule-like shadow spreading all over the two sides of the lung. Electrocardiogram revealed manifestations of acute pulmonary heart disease. The patient needed discrete inhalation of oxygen. Because the patient was resistant to chemotharepy drugs, his symptoms exacerbated increasingly. The later chest radiograph suggested pneumothorax, with right lung compressed by 50 %, and left lung by 40 %. After treatment with right deflation by 800 ml, chest radiograph still showed a large amount of air. The patient was then treated by intravenous injection (once every three days and totally 4 times) of 20 mg activated carbon agent of the present invention together with 20 mg DDP and 0.25 g 5-Fu. One week after the last injection, chest radiograph this time appeared normal and the trapped air was absorbed entirely.

### Case 3:

A female patient, aged 42, was diagnosed with right nasopharygeal T-cell Non-Hodgkins' lymphoma. After systemic chemotherapy and nasopharygeal chemotherapy successively, right cervical lymph node measured 1 x1 cm still existed. The patient was then subject to intravenous injection of 20 mg activated carbon agent of the present invention. Two days later, the above mentioned lymph node disappeared. She is in stable condition at present and there are no other treatments in progress.

### Case 4:

Another female patient, aged 38 years old, was diagnosed with breast cancer with systematic metastasis. She had difficulty in walking. Morphine was of no effect for analgesia. By way of intravenous injection of 20 mg activated carbon agent of the present invention, pain was distinctively eased after 7 hours of the treatment. She was able to freely walk after 24 hours of the treatment, and gained weight by 2 kg and can do some routine housework after three month of the treatment.

### Case 5:

Still another male patient, aged 67 years old, suffered successively from schistosom-induced hepatocirrhosis, chronic hepatitis B, diabetes, alcoholic hepatocirrhosis, pulmonary tuberculosis. He once was admitted due to hemoptysis and was diagnosed with right superior pulmonary cancer and there was no chance for operation, chemotherapy and radiotherapy because of agedness and sickliness. He then underwent intravenous injection of 20 mg activated carbon agent of the present invention. After 24 hours, hemoptysis was obviously mitigated. In the end of ten days' continuous administration, symptoms were gotten rid of. Supported by chest radiograph and compared with that of the previous, shadow of lump shrank relatively with clear-cut border. No marked changes happened to the focus. At present, this patient is in stable condition and there are no other treatments in progress.

### Examples

The purpose of embodiments of the present invention specifically disclosed below is just to illustrate the foregoing invention more clearly, rather than to limit the application of the present invention to the scope of the examples in the following description.

### Example 1:

### Preparation of activated carbon particles for injection:

500g activated carbon with a specific surface area of 6800 M²/g was physically ground into 120 mesh fine powder followed by input to a jet pulverizer for further smashing. The diameter of the resulted particles was controlled between 35 µm - 2 nm, with particles size mainly distributed in a range of 6 µm - 2 nm, wherein those between 3 µm - 2 nm account for 99 %, those between 6 µm - 3 µm no more than 1 %, and those with largest size between 35 µm - 6 µm are counted no more than 2 x 10⁴/mg. Using the above mentioned activated carbon particles as solute and dimethyl sulfoxide (DMSO) as suspension solvent, 5000 ml activated carbon suspension with concentration of 1 % was prepared. After stirred by strong magnetic force, the suspension was bottled (1 ml or 2 ml) respectively for use of transfusion injection.

### Example 2:

### Preparation of activated carbon particles injection:

100 g activated carbon with a specific surface area of 6800 M²/g was physically ground into 120 mesh fine powder followed by introduction to 300 ml thionyl chloride/chloroform solution with concentration of 1% and controlled temperature at -10°C. The system was stirred for 2 hours to enable adsorption reaction and then centrifuged at 20,000 rpm. Next, the container, with the sediment within it, was put into a pressure container. Heated at 80°C for 5 minutes, the pressure container was decompressed. Thus oxidized activated carbon was made. Afterwards, different activators may be introduced to the pressure container to produce different activated carbons carrying different active groups.

In case of preparation of hydroxyl activated carbon, water vapor at 90°C was introduced for 2 hours. After decompression, hydroxyl activated carbon would be produced;

In case of preparation of imino activated carbon, pure ammonia vapor at 90°C was input for 2 hours. After decompression, imino activated carbon would be produced;

In case of preparation of hydroxyl, amino activated carbon, 10-40 % ammonia-water vapor at 90°C was introduced for 2 hours. After decompression, activated carbon bearing both of hydroxyl and amino would be produced;

In case of preparation of carbo-hydro activated carbon, 100 g activated carbon with a specific surface area of 6800 M²/g was physically ground into 120 mesh fine powder followed by introduction of LiAlH₄ dissolved in aether. In this way, 300 ml solution with concentration of 1 % was prepared. The system was stirred for 2 hours to enable adsorption reaction and then centrifuged. After heated in a pressure container at 95°C for 5 minutes, the pressure container was decompressed. Thus carbo-hydro and lithium activated carbon was made.

The above mentioned activated carbon was input to a jet pulverizer for further smashing. The diameter of the resulted particles was controlled between 35 µm -2 nm, with particles size mainly distributed in a range of 6 µm - 2 nm, wherein those between 3 µm - 2 nm account for 99 %, those between 6 µm - 3 µm no more than 1 %, and those with largest size between 35 µm - 6 µm are counted no more than 2 x 10⁴/mg activated carbon micropowder; and was bottled (containing 10 mg or 20 mg activated carbon) respectively for use of transfusion injection.

### Example 3:

Preparation of drug-delivery activated carbon injection:
(1) Injection adsorbed with anticancer drugs: under circumstance of no antagonism exists, any drugs can be used for adsorption, for example, 6 g cisplatin and 0.4 g 5-FU, or 6 g cisplatin and 0.8 g 6-MP were dissolved in 100 ml water. Then 10 g activated carbon of 120 mesh or enabled hydroxyl-amino activated carbon was added to the obtained solution. The system was then stirred in order to have the adsorption reaction complete. Sediment obtained by high-speed centrifugation was dehydrated by acetone. Afterwards, the processed sediment was dipped into 0.1 % chitosan aqueous solution for 5 minutes followed again by acetone dehydration and desiccation. The resulted sediment was ground by a jet pulverizer. The diameter of the resulted particles was controlled between 35 µm - 2 nm, with particles size mainly distributed in a range of 6 µm - 2 nm, of which those between 3 µm - 2 nm account for 99 %, those between 6 µm - 3 µm no more than 1 %, and those with largest size between 35 µm - 6 µm are counted no more than 2 x 10⁴/mg. The treated drug-delivery activated carbon particles, with newly exposed sections after smashing, was dipped into 20 % ferroporphyrin acetone solution for 10 minutes. Sediment was obtained by high-speed centrifugation and desiccation. Then the final substance was bottled (containing 10 mg or 20 mg activated carbon) respectively for use of transfusion injection.
   The measured adsorption values of some drugs are as following: the adsorption values of activated carbon / hydroxyl-amino carbon with a specific surface area of 6800 M²/g were determined as (values of activated carbon (mg) /those of hydroxyl-amino carbon (mg)): uracil 33/51, adenine 213/260, guanine 331/378, 5-chlorouracil 75/102, 5-fluorouracil 20/49, thymine 5/17, thymopeptide 90/127, 6-MP 220/304, cisplatin 995/1105, ferroporphyrin 283/311, CTX 98/136, chloroform 10/4.
(2) Injection adsorbed with anticoagulation and cholesterol-lowering drugs: for example, 100 mg r-tPA, or 80mg cholesterol acyltransferase, or 220 mg nicotinic acid was ground in a jet pulverizer into 1 g hydroxyl activated carbon with particles size less than 0.5 µm. After sufficient aqueous adsorption, sediment was obtained by high-speed centrifugation and acetone dehydration and desiccation. Afterwards, the processed sediment was dipped into 0.1 % chitosan aqueous solution for 5 minutes followed again by high-speed centrifugation, acetone dehydration and desiccation. The resulted substance was bottled (containing 10 mg activated carbon) respectively for use of transfusion injection, which can be used to dissolve embolism in case of cardiac, encephalic embolism and treat vascular sclerosis and hypertension, etc.
(3) Injection adsorbed with antibiotics: for example, 500 mg isoniazid, or 200 mg rifampicin was ground in a jet pulverizer into 1 g hydroxyl-amino activated carbon with particles size less than 0.7 µm. After aqueous adsorption for 20 minutes, sediment was obtained by high-speed centrifugation and acetone dehydration and desiccation. Afterwards, the processed sediment was dipped into 0.1 % chitosan aqueous solution for 5 minutes followed again by high-speed centrifugation, acetone dehydration and desiccation. The resulted substance was bottled (containing 20 mg activated carbon) respectively for use of transfusion injection, which can be used to treat intractable bone tuberculosis and miliary pulmonary tuberculosis.
(4) Injection adsorbed with drugs: 100 mg IFN-γ was ground in a jet pulverizer into 1g hydroxyl-amino activated carbon with particles size less than 0.3 µm. After sufficient aqueous adsorption, sediment was obtained by high-speed centrifugation and acetone dehydration and low-temperature desiccation. Afterwards, the processed sediment was dipped into serine, specific antihapten antibody orienting agent for 5 minutes followed again by high-speed centrifugation, acetone dehydration and low-temperature desiccation. The resulted substance was bottled (containing 10 mg or 20 mg activated carbon) respectively for use of transfusion injection, which can be used to treat various intractable diseases induced by virus infection, such as Guillian-Barre's syndrome caused by EB virus and virus encephalopathy, as well as AIDS.

## Claims

1. An activated carbon based intravenous transfusion agent for pure activated carbon particles injection, enabled activated carbon particles injection, activated carbon injection adsorbed with catalyst, and therapeutic drug-delivery activated carbon injection used for intravenous transfusion; the therapeutic drug-delivery activated carbon injections comprising those adsorbing anticancer drugs, anticoagulation and cholesterol-lowering drugs, antibiotics, and antivirus drugs, is **characterized in that** the raw material, activated carbon, used in preparation of this group of agents should be in accordance with the following requirements: the specific surface area greater than or equal to 400-10000 M²/g or even much larger , the content of heavy metals ≤0.1-10 ppm, other dissoluble metal ions ≤0.1-12 ppm, ash content < 2%, the overall pore volume > 0.3-6 cm³/g, 0.15 % methylene blue adsorption > 6-30; the diameter of the activated carbon particles contained in this activated carbon based transfusion agent is from 35 µm to 2 nm, with particles size mainly distributed in a range of 6 µm - 2 nm, of which those between 3 µm - 2 nm account for 99 %, those between 6 µm - 3 µm no more than 1 %, and those with largest size between 35 µm - 6 µm are counted no more than 2 x 10⁴/mg.

2. The preparation method of the activated carbon based transfusion agent of claim 1, wherein this method embodies the following steps:
1) The activated carbon powder is physically ground into coarse 120 mesh particles;
2) After coarse particles adsorbed the activator with concentration of 0.01-10 %, the activated carbon is activated; the reaction system is heated to 70-190°C in a high-pressure container for 5-20 minutes and the activated carbon is consequently oxidized or reduced followed by refrigeration of the system and discharge of the residue gas; then aqueous vapor and ammonia vapor with temperatures at 60-100°C are forced into the system respectively and activated carbon comprising carbo-hydroxyl group, carbo-imino group or carbo-hydro group are then produced;
3) As for drug-delivery treatment of the activated carbon, liquid saturated adsorption is adopted to treat the selected drugs with enabled activated carbon or non-enabled pure activated carbon; then it is desiccated under low temperature and smashed into activated carbon particles with desired particles size by a jet pulverizer;
4) The activated carbon particles is treated with dipping approach for adsorption of biological orienting agents, and then processed by rapid, deep-freeze desiccation.

3. The preparation method of the activated carbon based intravenous transfusion agent of claim 2, wherein the activator in step (2) can be divided into oxidant and reductant, of which oxidant can be selected from the group of epoxy aether, acryl chloride, anhydride, aldehyde, and reductant can be selected the group of LiAlH4 and NaBH₄.

4. The preparation method of the activated carbon based intravenous transfusion agent of claim 2, wherein the relevant remedies illustrated in step (3) include: 1) traditional anticancer drugs, such as 5-FU, CTX, MTX, Ara-C/A, 6-MP, anticancer antibiotics, cisplatin/carboplatin, arsenic, pacilitaxel, etc.; 2) immune activating and regulating cytokines, such as: IL-2, 4, 6, GM, G-CSF, γ-IFN, TNF, FCF-B, VECF, 4-IBB/4-IBBL, B7-1 B7-2, toad-poison protein, specific antihapten antibody, etc.; 3) catalysts, such as core metal complexes with benzyl/ butyl lithium group, and transition metals including cuprammonium agent, platinum, palladium agent, selenium, germanium compounds and so on; these compounds are featured with double functions of catalysis and cancer-killing; 4) adsorbent biological enzymes, such as oxidoreductases, transaminases, hydrolases and isomerases etc.

5. The preparation method of the activated carbon based intravenous transfusion agent of claim 2, wherein the referred biological orienting agents in step (4) include ferroporphyrin, or a large group of thymine cytosine, uracil adenine, guanine, and their derivatives, such as 5- fluorouracil, hydrodeuteradenine, methotrexate, as well as cancer cell adhesion factors (e.g., vascular cell adhesion molecule-1, VCAM-1) and cancer cell growth factors (e.g., vascular enthothelial growth factor, VEGF).

6. The use of the activated carbon based transfusion agent in the manufacture of anticancer drug of claim 1, which is **characterized by** effective therapeutic dose of 0.1-5 mg/ body weight per time of transfusion into blood, 1-7 times per week, the total average cumulative dose for adults less than 10 g.
